# EUROPEAN PATENT SPECIFICATION

(11) **EP 3 533 453 B1**
(45) Date of publication and mention of the grant of the patent: **09.04.2025**
(21) Application number: 17865766.4
(22) Date of filing: 30.10.2017
(51) Int. Cl.: A61K 35/744, A23L 33/135, A61K 35/747, A61P 1/08, A61P 27/02, A61P 27/16

(54) **AN AGENT COMPRISING A LACTIC ACID BACTERIUM FOR USE IN REDUCING THE RISK OF DEVELOPING AN EYE DISEASE OR EQUILIBRIUM DYSFUNCTION**
EIN MITTEL ENTHALTEND EIN MILCHSÄUREBAKTERIUM ZUR VERWENDUNG ZUR REDUZIERUNG DES RISIKOS DER ENTWICKLUNG EINER AUGENKRANKHEIT ODER EINER GLEICHGEWICHTSSTÖRUNG
AGENT COMPRENANT UNE BACTÉRIE D'ACIDE LACTIQUE DESTINÉ À ÊTRE UTILISÉ POUR RÉDUIRE LE RISQUE DE DÉVELOPPEMENT D'UNE MALADIE OCULAIRE OU D'UN DYSFONCTIONNEMENT DE L'ÉQUILIBRE

(30) Priority: 28.10.2016 JP 2016211510; 28.10.2016 JP 2016211511; 28.10.2016 JP 2016211512
(43) Date of publication of application: 04.09.2019
(73) Proprietor: Kabushiki Kaisha Yakult Honsha, Tokyo 105-8660 (JP); Tokyo Metropolitan Institute for Geriatrics and Gerontology, Tokyo 173-0015 (JP)
(72) Inventor: AOYAGI, Yukitoshi, Tokyo 173-0015 (JP); MATSUBARA, Satoshi, Tokyo 105-8660 (JP); HONDA, Yusuke, Tokyo 105-8660 (JP); AMAMOTO, Ryuta, Tokyo 105-8660 (JP); MIYAZAKI, Koji, Tokyo 105-8660 (JP)
(74) Representative: Wächtershäuser & Hartz Patentanwaltspartnerschaft mbB
(86) International application number: PCT/JP2017/039045
(87) International publication number: WO 2018/079760

(56) References cited:
- EP-A2- 2 429 557
- WO-A1-2007/133188
- WO-A1-2015/174481
- WO-A1-2016/136942
- WO-A1-2017/010520
- JP-A- 2003 073 286
- JP-A- 2007 126 399
- JP-A- 2011 254 743
- US-A1- 2006 177 424

## Description

### Technical Field

The present invention relates to an agent or a food that can inhibit the onset of overall disease by continuous ingestion.

### Background Art

The average lifespans of Japanese in 2014 are 86.83 years for women and 80.50 years for men, which both have reached the record highs, and have a possibility of continuing to grow with improvements in medical technology and increased health consciousness. On the other hand, the period of healthy daily life when one can live without being restricted due to health problems is called healthy lifespan. As the aging progresses, if the unhealthy period, which is the difference between the average lifespan and the healthy lifespan, expands, problems such as an increase in medical expenses and nursing care expenses due to suffering with diseases and an increase in burden on the family members of the patient occur. Accordingly, in order to inhibit the increase in medical expenses, etc. and to prevent deterioration of the quality of life of individuals, extending the healthy lifespan has become a challenge.

In order to extend the healthy lifespan, methods such as improvements in lifestyle and social environment are needed. In addition, preventive measures against various diseases are also effective, but no method for collectively inhibiting the onset of overall disease with different causes is known. Accordingly, a method for inhibiting the onset of overall disease in a simple manner and extending the healthy lifespan has been desired.

Eye diseases are diseases occurring in eyes that control visual sensation and include various diseases with different causes and symptoms. Examples of the eye diseases include cataract, glaucoma, age-related macular degeneration, fundus hemorrhage, and vitreous floater.

Cataract is a disease in which the crystalline lens becomes cloudy, and examples of the cause thereof include aging, congenital causes, external injury, atopy, agents, radiation, and other eye diseases. In cataract, the crystalline lens scatters light to cause symptoms, such as blurred vision, double vision, discomfort with bright light, and low vision. In the incipient stage of cataract, eye drops treatment for the purpose of preventing the progress is performed, but in the advanced stage of cataract, surgery is a common treatment.

Glaucoma is a disease in which the optic nerve has been impaired and is common in middle and old age, and the optic nerve impairment can usually be ameliorated or inhibited by sufficiently lowering the intraocular pressure. As symptoms of glaucoma, the occurrence of partial loss of vision and a narrowed visual field are common, and progression causes low vision and even blindness. Glaucoma is the leading cause of blindness in Japan. Since once impaired optic nerve cannot be recovered, treatment of glaucoma is aimed at stopping or inhibiting the progression, and examples of the treatment include drug therapy, laser therapy, and surgery.

Age-related macular degeneration is a disease in which the macula, which is the central part of the retina, is impaired due to aging. Damage of the macula causes symptoms, such as distortion of the central part of the visual field, loss of sight in the central part of the visual field, low vision, and blindness. Treatment, such as drug treatment, photodynamic therapy, laser coagulation, and surgery, is performed, and in recent years, clinical trials of therapy for age-related macular degeneration by transplantation of a retinal pigment epithelial cell sheet produced from artificial pluripotent stem cells (iPS cells) have been performed.

Vitreous floater is a symptom in which something like dirt or an insect that seems to be flying is seen in the visual field. Most cases are due to physiological changes such as aging and do not need to be treated, but cases due to diseases, such as retinal detachment, need to be treated.

Fundus hemorrhage is retinal hemorrhage caused by rupture or obstruction of a blood vessel on the retinal surface due to a disease such as diabetes or retinal vein occlusion or an external injury. The main symptoms are low vision, floater, etc., and low vision occurs when the hemorrhage occurs at the central part. In fundus hemorrhage by a systemic disease, it is important to treat the primary disease, and in addition, drug treatment or laser treatment is also additionally performed.

As the aging society proceeds, these eye diseases are concerned to further increase, and symptoms, such as low vision, due to eye diseases may be a major impediment in daily life. Regarding the prevention of eye diseases, methods such as improvement of lifestyle and reduction of excessive burden on eyes are suggested, but there is no known method for reducing the incidence of eye diseases in general which have different causes and symptoms, and it has been desired to develop a method for reducing the incidence of eye diseases in a simple manner.

Humans have the function of sensing changes in body movement and direction of gravity, called the equilibrium sense. The balance of the body is maintained by integrating three types of sensation information of vestibular sensation, visual sensation, and deep sensation in the central nervous system and outputting motion information based on the information to limbs and trunk muscles and so on.

Diseases in which the equilibrium sense is impaired by some abnormality in organs of the body cooperatively working to maintain the equilibrium sense are collectively referred to as equilibrium dysfunction here. Possible causes of equilibrium dysfunction include impairment of vestibular organs that are receptors of vestibular sensation and of vestibular nerves that are transmission paths, and abnormalities of the central nervous system, such as the brain stem and the cerebellum. Specifically, examples of the equilibrium dysfunction include dizziness syndrome and Ménière's disease.

The term dizziness syndrome refers to symptoms of dizziness in which, although dizziness, such as vertigo, which is the sensation that the surroundings seem to be moving and dizziness, which is a sensation of floating or unsteadiness of the body, is complained of, the cause of the dizziness is not clearly identified. For alleviating or treating the symptoms, for example, massage and drug therapy are applied.

Ménière's disease is a disease caused by endolymphatic hydrops of the inner ear, causing severe vertigo and symptoms of hearing loss, tinnitus, and fullness of the ear, and repeating them. The treatment is basically drug therapy, and in some refractory or severe cases, surgery is performed.

If the equilibrium sense is impaired, it is difficult to maintain a normal physiological posture, and accidents including a fall accident are also caused. In addition, symptoms such as dizziness and wobble may be major impediments in daily life. As the prevention of equilibrium dysfunction, improvement of lifestyle, such as exercise, is suggested, but there is no known method for inhibiting the onset of equilibrium dysfunction in general which have different causes and symptoms, and it has been desired to develop a method for inhibiting the onset of equilibrium dysfunction in a simple manner.

On the other hand, regarding lactic acid bacteria, for example, regulatory effect on the functions of the intestines and immunoregulatory effect are known. Many human clinical trials using Lactobacillus casei, one of lactic acid bacteria, have been conducted, for example, improvement of intestinal flora and prevention of infection (Non Patent Literature 1), improvement of fecal property (Non Patent Literatures 2 and 3), prevention of cancer recurrence (Non Patent Literature 4), and immunoregulatory effect (Non Patent Literature 5) have been reported. In addition, epidemiological studies have reported inhibitory effects on the onset of breast cancer and bladder cancer by ingestion of lactic acid bacteria beverages containing Lactobacillus casei (Non Patent Literatures 6 and 7). However, the epidemiologic preventive effect on overall disease is unclear.

### Citation List

### Non Patent Literature

Non Patent Literature 1: Gleeson M., Bishop N.C., Oliveira M., and Tauler P., Daily probiotic's (Lactobacillus casei Shirota) reduction of infection incidence in athletes, Int. J. Sport. Nutr. Exerc. Metab., 2011, 21(1): 55-64
Non Patent Literature 2: Sur D., Manna B., Niyogi S.K., Ramamurthy T., Palit A., Nomoto K., Takahashi T., Shima T., Tsuji H., Kurakawa T., Takeda Y., Nair G.B., and Bhattacharya S.K., Role of probiotic in preventing acute diarrhoea in children: a community-based, randomized, double-blind placebo-controlled field trial in an urban slum, Epidemiol. Infect., 2011, 139(6): 919-26
Non Patent Literature 3: Sakai T., Makino H., Ishikawa E., Oishi K., and Kushiro A., Fermented milk containing Lactobacillus casei strain Shirota reduces incidence of hard or lumpy stools in healthy population, Int. J. Food Sci. Nutr., 2011, 62(4): 423-30
Non Patent Literature 4: Aso Y., Akaza H., Kotake T., Tsukamoto T., Imai K., and Naito S., Preventive effect of a Lactobacillus casei preparation on the recurrence of superficial bladder cancer in a double-blind trial. The BLP Study Group, Eur. Urol., 1995, 27(2): 104-9
Non Patent Literature 5: Nagao F., Nakayama M., Muto T., and Okumura K., Effects of a fermented milk drink containing Lactobacillus casei strain Shirota on the immune system in healthy human subjects, Biosci. Biotechnol. Biochem., 2000, 64(12): 2706-8
Non Patent Literature 6: Toi M., Hirota S., Tomotaki A., Sato N., Hozumi Y., Anan K., Nagashima T., Tokuda Y., Masuda N., Ohsumi S., Ohno S., Takahashi M., Hayashi H., Yamamoto S., and Ohashi Y., Probiotic Beverage with Soy Isoflavone Consumption for Breast Cancer Prevention: A Case-control Study, Curr. Nutr. Food Sci., 2013, 9(3): 194-200
Non Patent Literature 7: Ohashi Y., Nakai S., Tsukamoto T., Masumori N., Akaza H., Miyanaga N., Kitamura T., Kawabe K., Kotake T., Kuroda M., Naito S., Koga H., Saito Y., Nomata K., Kitagawa M., and Aso Y. Habitual intake of lactic acid bacteria and risk reduction of bladder cancer. Urol. Int., 2002, 68(4): 273-80
   WO 2007/133188 A1 and US 2006/177424 A1 disclose a method for treatment or prophylaxis of a disease state or other physiological condition by administration of an antifungal composition that includes at least one of the bacilli (1) Bacillus subtilis, (2) Lactobacillus sporogenes, and (3) Streptococcus faecalis.
   JP 5185976 B2 discloses a composition for improving diabetes symptoms and complications thereof.
   WO 2015/174481 A1 discloses an agent for the prevention and treatment of chlamydia infection.
   JP 2003 073286 A discloses an agent for the prevention and treatment of inflammatory bowel disease.
   WO 2016/136942 A1 discloses a colitis inhibitor.
   JP 2007 126399 A discloses a composition for oral ingestion capable of increasing in vivo glutathione concentration.
   EP 2429557 A2 discloses infant feeding formulas comprising probiotic micro-organisms.

### Summary of the Invention

### Technical Problem

An object of the present invention is to provide an agent or a food or drink for inhibiting the onset of overall disease and extending the disease-free period without disease onset, but not for treating the disease that has occurred, by ingestion before the onset of the disease. Specifically, an object of the present invention is to provide an agent or a food or drink for reducing the risk of developing eye diseases, but not for treating the eye diseases that have occurred, by ingestion before the onset of the eye diseases. In addition, specifically, an object of the present invention is to provide an agent or a food or drink for reducing the risk of developing equilibrium dysfunction, but not for treating the equilibrium dysfunction that has occurred, by ingestion before the onset of the equilibrium dysfunction.

### Solution to Problem

Accordingly, the present inventors investigated the relationship between the ingestion of lactic acid bacteria and the disease-free rate showing the proportion of individuals who do not have the onset of diseases and found that the decrease with time in a disease-free rate in individuals who have habitually taken lactic acid bacteria is significantly inhibited compared to the decrease with time in a disease-free rate in individuals who do not habitually take lactic acid bacteria. The present inventors also investigated the relationship between the ingestion of lactic acid bacteria and the incidence of eye diseases and found that the incidence of eye diseases in humans who have habitually taken lactic acid bacteria is decreased compared to the incidence of eye diseases in humans who do not habitually take lactic acid bacteria. Furthermore, the present inventors investigated the relationship between the ingestion of lactic acid bacteria and the incidence of equilibrium dysfunction and found that the incidence of equilibrium dysfunction in humans who have habitually taken lactic acid bacteria is decreased compared to the incidence of equilibrium dysfunction in humans who do not habitually take lactic acid bacteria. Consequently, the present invention has been accomplished.

The present invention relates to the subject matter of claims 1 to 4. That is, the present invention provides an agent comprising a lactic acid bacterium as an active ingredient for use in reducing the risk of developing an eye disease selected from the group consisting of age-related macular degeneration, fundus hemorrhage, and vitreous floater, or dizziness syndrome as an equilibrium dysfunction;
wherein the agent inhibits a decrease in a disease-free rate, and
wherein the lactic acid bacterium is Lactobacillus casei YIT 9029 (FERM BP-1366).The present invention also provides a food or drink or a food or drink composition comprising a lactic acid bacterium as an active ingredient for use in reducing the risk of developing an eye disease selected from the group consisting of age-related macular degeneration, fundus hemorrhage, and vitreous floater, or dizziness syndrome as an equilibrium dysfunction;
   wherein the food or drink or the food or drink composition inhibits a decrease in a disease-free rate, and
wherein the lactic acid bacterium is Lactobacillus casei YIT 9029 (FERM BP-1366).

### Effects of the Invention

According to the present invention, the onset of overall disease can be inhibited by ingestion of lactic acid bacteria, and the disease-free period without disease onset can be extended. In addition, the risk of developing eye diseases can be reduced by ingestion of lactic acid bacteria. Furthermore, the risk of developing equilibrium dysfunction can be reduced by ingestion of lactic acid bacteria.

### Brief Description of Drawings

[Figure 1] Figure 1 is a disease-free rate curve formed by a Kaplan-Meier method (for 5 years).
[Figure 2] Figure 2 is a disease-free rate curve formed by a Kaplan-Meier method (for 10 years).

### Description of Embodiments

The agent for the use of the invention inhibits a decrease in a disease-free rate and can be used for reducing risk of developing an eye disease or for reducing risk of developing equilibrium dysfunction. The present invention relates to an agent for use in reducing risk of developing an eye disease, or for reducing risk of developing equilibrium dysfunction , comprising a lactic acid bacterium as an active ingredient, and the lactic acid bacterium is Lactobacillus casei YIT 9029 (FERM BP-1366), and preferably living Lactobacillus casei YIT 9029 (FERM BP-1366).

The mode of utilization of lactic acid bacteria used in the present invention is not particularly limited, and the lactic acid bacteria may be lyophilized or spraydried or may be used as a culture containing the bacteria or a treated product of the bacteria and may be used in any mode. The lactic acid bacterium is Lactobacillus casei YIT 9029 (FERM BP-1366).

As shown in Examples below, the lactic acid bacterium has an effect of inhibiting a decrease in a disease-free rate. Accordingly, the lactic acid bacterium can function as an agent that inhibits a decrease in a disease-free rate and also can be used for producing an agent that inhibits a decrease in a disease-free rate. That is, the lactic acid bacterium can be used for inhibiting the onset of overall disease by ingestion of the bacterium by humans before the onset of the disease to extend the disease-free period without disease onset.

Here, the term "disease" refers to a disease that occurs in the human body and specifically is a concept including the diseases shown in Table 1 provided in Examples described below and diseases that are not shown in Table 1 but diagnosed by a doctor.

The term "disease-free" refers to a condition free from the onset of diseases, and the term "disease-free rate" refers to the proportion of individuals who do not develop the above-mentioned diseases in a population. According to the present invention, the decrease with time in a disease-free rate can be inhibited by habitual ingestion of lactic acid bacteria 3 or more times per week, compared to the case in which the bacteria are taken only less than 3 times per week.

As shown in Examples described below, the subjects "without onset" shown in Table 2 are also free from the onset of eye diseases such as cataract and glaucoma, and it is therefore suggested that the risk of developing eye diseases is also reduced. Furthermore, the subjects "without onset" shown in Table 2 are also free from the onset of equilibrium dysfunction such as dizziness, and it is therefore suggested that the risk of developing equilibrium dysfunction is also reduced.

As shown in Examples described below, the lactic acid bacterium has an effect of reducing risk of developing eye diseases. Accordingly, the lactic acid bacterium can function as an agent for reducing risk of developing an eye disease and also can be used for producing an agent for reducing risk of developing an eye disease. That is, the lactic acid bacterium can be used for reducing the risk of developing eye diseases by ingestion of the bacterium by humans before the onset of eye diseases.

Here, the term "eye disease" is age-related macular degeneration, fundus hemorrhage, or vitreous floater.

The term "reduction of risk of developing a disease" refers to prevention or delay of disease onset. According to the present invention, the risk of developing eye diseases can be reduced by habitual ingestion of lactic acid bacteria 3 or more times per week, compared to the case in which the bacteria are taken less than 3 times per week.

As shown in Examples described below, the lactic acid bacterium has an effect of reducing risk of developing equilibrium dysfunction. Accordingly, the lactic acid bacterium can function as an agent for reducing risk of developing equilibrium dysfunction and also can be used for producing an agent for reducing risk of developing equilibrium dysfunction. That is, the lactic acid bacterium can be used for reducing the risk of developing equilibrium dysfunction by ingestion of the bacterium by humans before the onset of equilibrium dysfunction.

Here, the "equilibrium dysfunction" is a disease occurring in an organ that contributes to maintain the equilibrium sense and impairs the equilibrium sense of the body, namely dizziness syndrome. The "dizziness syndrome" shows symptoms such as vertigo, which is the sensation that the surroundings seem to be moving, and dizziness, which is a sensation of floating or unsteadiness of the body, whose cause is not clearly identified.

The term "reduction of risk of developing a disease" refers to prevention or delay of disease onset. According to the present invention, the risk of developing equilibrium dysfunction can be reduced by habitual ingestion of lactic acid bacteria 3 or more times per week, compared to the case in which the bacteria are taken less than 3 times per week.

The agent inhibiting a decrease in a disease-free rate and so on used in the present invention can be used either orally or parenterally, but oral administration is preferred. Upon administration, a composition containing lactic acid bacteria, preferably living lactic acid bacteria, as an active ingredient is mixed with a solid or liquid pharmaceutical nontoxic carrier suitable for an administration method, such as oral administration, intrarectal administration, or injection, and can be administered in the form of a common pharmaceutical preparation. Examples of the preparation include solid preparations, such as a tablet, a granule, a powder, and a capsule; liquid preparations, such as a solution, a suspension, and an emulsion; and lyophilized preparations. These preparations can be prepared according to a common formulation method. Examples of the pharmaceutical nontoxic carrier include glucose, lactose, sucrose, starch, mannitol, dextrin, fatty acid glyceride, polyethylene glycol, hydroxyethyl starch, ethylene glycol, polyoxyethylene sorbitan fatty acid ester, amino acid, gelatin, albumin, water, and saline. In addition, commonly used additives, such as a stabilizer, a moisturizer, an emulsifier, a binder, a tonicity agent, and an excipient, can be appropriately added, as necessary.

As the pharmaceutical preparation, a commercially available preparation may be used, and a pharmaceutical preparation containing living Lactobacillus casei manufactured by Yakult Honsha Co., Ltd. can be suitably used. Examples of the preparation include "Yakult BL Intestinal Regulator" and "Yakult BL Intestinal Regulator S-Tablet". As a pharmaceutical product for medical use, "Biolactis Powder" is available.

The agent inhibiting a decrease in a disease-free rate and so on used in the present invention can be used not only as the above-described preparations but also as a food or drink containing lactic acid bacteria, preferably living lactic acid bacteria. That is, the agent inhibiting a decrease in a disease-free rate and so on used in the present invention can be used as a food or drink for inhibiting a decrease in a disease-free rate, a food or drink for reducing risk of developing an eye disease, or a food or drink for reducing risk of developing equilibrium dysfunction that contains a lactic acid bacterium as an active ingredient. In addition, the agent inhibiting a decrease in a disease-free rate and so on used in the present invention can be used as a food or drink composition inhibiting a decrease in a disease-free rate, a food or drink composition for reducing risk of developing an eye disease, or a food or drink composition for reducing risk of developing equilibrium dysfunction that contains a lactic acid bacterium as an active ingredient. Here, when lactic acid bacteria, preferably living lactic acid bacteria, are added to a food or drink, the food or drink may contain the bacteria as such or together with various nutrients. The composition used in the present invention can be used as a health care food or food material useful for inhibition of the onset of overall disease, i.e., extension of the disease-free period, specifically, as a health care food or food material useful for inhibition of the onset of eye diseases or equilibrium dysfunction. Indication of the above-described effects may be attached to these foods or drinks or containers thereof. Specifically, when lactic acid bacteria, preferably living lactic acid bacteria, are blended with a food or drink, the bacteria may be formed into a form suitable for foods through a common method by appropriately using additives usable as foods or drinks, that is, may be formed into, for example, a granule, a grain, a tablet, a capsule, or a paste, or may be used by being added to various foods, for example, meat processed food such as ham and sausage, processed sea food such as kamaboko (boiled fish paste) and chikuwa (tubular fish paste), bread, confectionery, butter, or powdered milk, or may be used by being added to beverages, for example, water, fruit juice, milk, soft drink, and a tea beverage.

Furthermore, as the food or drink, a fermented food or drink, such as fermented milk food or drink, fermented soymilk, fermented juice, and fermented vegetable juice, containing lactic acid bacteria, preferably living lactic acid bacteria, can be suitably used, and the use of a fermented milk food or drink is particularly preferred. The fermented milk food or drink may be produced according to an ordinary method. For example, in the case of producing fermented milk, lactic acid bacteria are inoculated and cultured alone or together with another microorganism in a sterilized milk medium and is homogenized to obtain a fermented milk base. Subsequently, a separately prepared syrup solution may be added to and mixed with the base, and after homogenization with, for example, a homogenizer, a flavor may be further added thereto to obtain the final product. The thus-obtained fermented milk food or drink may be formed into any form such as a plain type not containing syrup (sweetener), a soft type, a fruit flavor type, a solid form, or a liquid form.

Such a fermented milk food or drink can be blended with arbitrary ingredients, such as a sweetener such as syrup, an emulsifier, a thickener (stabilizer), and various vitamins. Examples of the syrup include saccharides, such as glucose, sucrose, fructose, fructose glucose liquid sugar, glucose fructose liquid sugar, palatinose, trehalose, lactose, xylose, maltose, honey, and molasses; sugar alcohols, such as sorbitol, xylitol, erythritol, lactitol, palatinit, reduced starch syrup, and reduced maltose syrup; and high-intensity sweeteners, such as aspartame, thaumatin, sucralose, acesulfame K, and stevia. The fermented milk food or drink may also be blended with an emulsifier, such as sucrose fatty acid ester, glycerin fatty acid ester, polyglycerin fatty acid ester, sorbitan fatty acid ester, and lecithin; and a thickener (stabilizer), such as agar, gelatin, carrageenan, guar gum, xanthan gum, pectin, locust bean gum, gellan gum, carboxymethyl cellulose, soybean polysaccharides, and propylene glycol arginate. In addition to these ingredients, the food or drink can also be blended with vitamins, such as vitamin A, B vitamins, vitamin C, and E vitamins; minerals, such as calcium, magnesium, zinc, iron, and manganese; an acidifier, such as citric acid, lactic acid, acetic acid, malic acid, tartaric acid, and gluconic acid; milk fat, such as cream, butter, and sour cream; a flavor, such as a yogurt, berry, orange, quince, perilla, citrus, apple, mint, grape, or apricot type flavor, pear, custard cream, peach, melon, banana, a tropical or herbal flavor, tea, and coffee; herbal extract; and brown sugar extract.

As the fermented milk food or drink, a commercially available product may be used, and a fermented milk food or drink containing living Lactobacillus casei manufactured by Yakult Honsha Co., Ltd. can be suitably used. Examples of such a food or drink include Yakult products such as "Yakult", "Yakult 300V", "Yakult SHEs", and "Yakult 400", "Joie", "Sofuhl", "Purela", "Pretio", and "Nyusankin Soy α", and the Yakult products containing a large number of living cells of Lactobacillus casei is particularly preferably used. In the present invention, the food or drink composition is a food or drink excluding plants and animals.

The ingestion amount of the lactic acid bacteria to be used as an active ingredient of the present invention is not strictly restricted, but its suitable ingestion amount is 1×10⁵ to 1×10¹³ cfu, particularly preferably 1×10⁸ to 1×10¹² cfu, per day as the number of living lactic acid bacteria. From the viewpoint of capable of effectively inhibiting a decrease with time in a disease-free rate, a higher frequency of ingestion of the lactic acid bacteria is preferred, and 3 or more times per week is particularly preferred. In addition, a longer period of ingestion of the lactic acid bacteria is preferred, and continuous ingestion for 5 or more years, in particular 10 or more years, is more preferred.

The agent for inhibiting a decrease in a disease-free rate and so on used in the present invention may contain another active ingredient as long as the effects of the present invention are not impaired.

Ingestion of the agent for inhibiting a decrease in a disease-free rate used in the present invention before disease onset can inhibit the onset of overall disease and can prevent the onset of overall disease.

Ingestion of the agent for reducing risk of developing an eye disease used in the present invention before eye disease onset can inhibit the onset of eye diseases and can prevent the onset of eye diseases. In addition, even after eye disease onset, the recurrence rate of the eye disease after treatment of the eye disease, such as surgery, medication, or laser treatment, can be reduced by ingestion of the agent for reducing risk of developing an eye disease of the present invention.

Ingestion of the agent for reducing risk of developing equilibrium dysfunction used in the present invention before equilibrium dysfunction onset can inhibit the onset of equilibrium dysfunction and can prevent the onset of equilibrium dysfunction. In addition, even after the onset of equilibrium dysfunction, the recurrence rate of the equilibrium dysfunction after treatment of the equilibrium dysfunction, such as medication or surgery, can be reduced by ingestion of the agent for reducing risk of developing equilibrium dysfunction of the present invention.

### Examples

The present invention will be described in further detail with reference to an example, but is not limited thereto at all.

### Example

Retrospective epidemiologic survey by an interview method and a self-recording method was performed for individuals aged 65 years and older who have physical functions that do not interfere with daily life and agreed with and signed the research content. The anamneses, the onset ages thereof, and the state of ingestion of a Lactobacillus casei-containing composition were investigated.

### 1. Anamnesis

The anamneses and the onset ages of subjects were investigated using the disease name code table shown in Table 1. Diseases not listed in the disease name code table were investigated by a method specifically describing the anamneses and the onset ages (free description). That is, age-related macular degeneration, fundus hemorrhage, and vitreous floater shown in Table 4, dizziness syndrome shown in Tables 5 and 6, and Ménière's disease shown in Table 6 are diseases investigated by free description.

**[Table 1]**

| Code No. | Disease Name | Code No. | Disease Name | Code No. | Disease Name |
|---|---|---|---|---|---|
| 7 | Hypertension | 320 | Lung cancer | 640 | Endometriosis |
| 400 | Hyperlipidemia | 170 | Gastric ulcer | 381 | Uterine myoma |
| 401 | Diabetes | 171 | Duodenal ulcer | 336 | Uterine cancer |
| 402 | Hyperuricemia | 300 | Gastric cancer | 392 | Ovarian cancer |
| 403 | Thyroid disease | 200 | Fatty liver | 566 | Urinary calculus |
| 1 | Subarachnoid hemorrhage | 201 | Hepatitis | 550 | Chronic renal failure |
| 3 | Cerebral hemorrhage | 202 | Hepatic cirrhosis | 567 | Dialysis |
| 4 | Cerebral infarction | 311 | Hepatic cancer | 850 | Cataract |
| 733 | Dementia | 250 | Anal disease | 878 | Glaucoma |
| 748 | Depression | 260 | Colorectal polyp | 963 | Osteoarthritis |
| 780 | Transient ischemic attack | 308 | Colorectal cancer | 967 | Rheumatoid arthritis |
| 5 | Arrhythmia | 280 | Cholecystitis | 968 | Gout |
| 8 | Angina pectoris | 380 | Gallbladder polyp | 978 | Collagen disease |
| 9 | Myocardial infarction | 290 | Pancreatic disease | 992 | Lower back pain |
| 811 | Congenital heart disease | 500 | Anemia | 993 | Osteoporosis |
| 120 | Pulmonary emphysema | 590 | Prostatic hypertrophy | 761 | Sleep apnea |
| 130 | Bronchial asthma | 340 | Prostatic cancer | | |
| 140 | Pneumonia | 600 | Mastopathy | | |
| 150 | Pulmonary tuberculosis | 330 | Breast cancer | | |

### 2. Ingestion state of Lactobacillus casei-containing composition

"Yakult", "Yakult LT", "Yakult 400", "Yakult 400LT", "Yakult 300", "Yakult 300V", "Yakult 200", "Yakult Ace", "Yakult 80Ace", "Yakult Fruity", "New Yakult", and "New Yakult Calorie Half", (hereinafter, referred to as "Yakults", manufactured by Yakult Honsha Co., Ltd.).

"Joie, Pretio, Sofuhl containing Lactobacillus casei", (manufactured by Yakult Honsha Co., Ltd.).

The above-mentioned products (Yakult, Joie, Pretio, and Sofuhl) all contain living cells of Lactobacillus casei YIT 9029.

The ingestion state (ingestion frequency) of the above-mentioned products by each subject over the past 10 years was investigated every 5 years, from 5 years ago to now and from 10 years ago to 5 years ago.

### 3. Analysis subject

When the investigation period was set to 5 years, subjects were divided into those who took a Lactobacillus casei-containing composition in the past 5 years less than 3 times per week (consumers of less than 3 times per week) and those who took a Lactobacillus casei-containing composition in the past 5 years 3 or more times per week (consumers of 3 or more times per week). Separately, when the investigation period was set to 10 years, subjects were divided into consumers of 3 or more times per week who took a Lactobacillus casei-containing composition 3 or more times per week in both periods of from 5 years ago to now and of from 10 years ago to 5 years ago and consumers of less than 3 times per week who were other than the consumers of 3 or more times per week. In the analysis of disease-free rate, subjects who had any anamnesis before the investigation period (5 years ago or 10 years ago) were excluded from the analysis subjects; in the analysis of risk of developing eye diseases, subjects who already had onset of any of eye diseases before the investigation period (10 years ago) were excluded; and in the analysis of risk of developing equilibrium dysfunction, subjects who already had onset of any of equilibrium dysfunction before the investigation period (5 years ago or 10 years ago) were excluded.

### 4. Definition of disease-free rate

The term disease-free rate refers to the percentage of subjects who did not have onset of any of the diseases shown in the disease name code table of Table 1 and the questionnaire among the analysis subjects.

### 5. Analysis method

In the analysis of disease-free rate, the analysis subjects were classified depending on two attributions: the ingestion frequency of a Lactobacillus casei-containing composition (ingestion less than 3 times per week or ingestion 3 or more times per week) and the presence or absence of disease onset. Table 2 shows the results when the investigation period was 5 years, and Table 3 shows the results when the investigation period was 10 years.

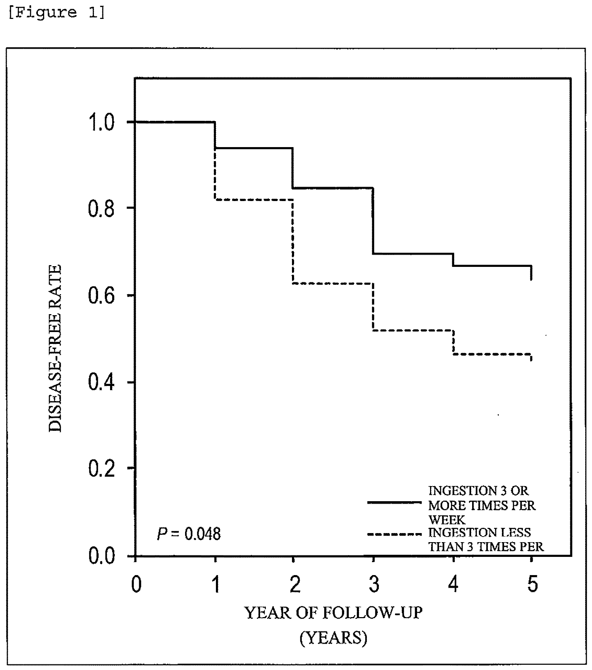

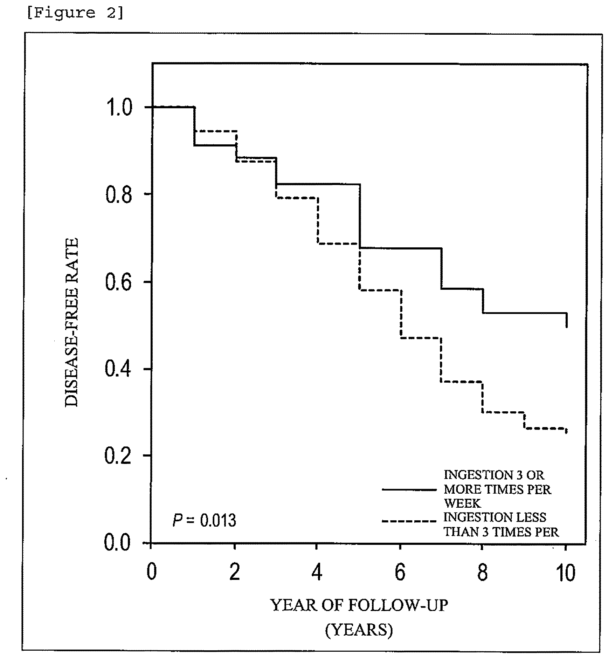

Disease-free rate curves of the two groups: the consumers of less than 3 times per week and the consumers of 3 or more times per week of a Lactobacillus casei-containing composition, were drawn by the Kaplan-Meier method, and whether a significant difference was present or not between the disease-free rates of the two groups was investigated by the Log-Rank test. It was determined there was a significant value at P value < 0.05. Figure 1 shows the results when the investigation period was 5 years, and Figure 2 shows the results when the investigation period was 10 years.

**[Table 2]**

| Relationship between the ingestion of Lactobacillus casei-containing composition 3 or more times per week for 5 years and the onset of overall disease | | | |
|---|---|---|---|
| Number of subjects (%) | | | |
| | Lactobacillus casei-containing composition | | |
| | Ingestion less than 3 times per week | Ingestion 3 or more times per week | Total |
| Without onset | 40 (44.9) | 21 (63.6) | 61 |
| Onset | 49 (55.1) | 12 (36.4) | 61 |
| Total | 89 (100) | 33 (100) | 122 |

**[Table 3]**

| Relationship between the ingestion of Lactobacillus casei-containing composition 3 or more times per week for 10 years and the onset of overall disease | | | |
|---|---|---|---|
| Number of subjects (%) | | | |
| | Lactobacillus casei-containing composition | | |
| | Ingestion less than 3 times per week | Ingestion 3 or more times per week | Total |
| Without onset | 43 (25.7) | 17 (50) | 60 |
| Onset | 124 (74.3) | 17 (50) | 141 |
| Total | 167 (100) | 34 (100) | 201 |

In the analysis of risk of developing eye diseases, among the analysis subjects, the number of subjects who had onset of any of eye diseases during the investigation period was defined as the number of patients. The percentage of patients in each group was defined as incidence. Furthermore, the value obtained by subtracting the incidence in the consumers of 3 or more times per week from the incidence in the consumers of less than 3 times per week was calculated as a change in the incidence by ingestion of a Lactobacillus casei-containing composition. Table 4 collectively shows the respective results.

**[Table 4]**

| Relationship between the ingestion of Lactobacillus casei-containing composition 3 or more times per week for 10 years and the incidence of eye diseases | | | | | | |
|---|---|---|---|---|---|---|
| | | Number of patients (subjects) | | Incidence (%) | | |
| | Number of analysis subjects (subjects) | Ingestion less than 3 times per week | Ingestion 3 or more times per week | Ingestion less than 3 times per week | Ingestion 3 or more times per week | Change in incidence by ingestion (%) |
| Cataract | 589 (3 or more times per week: 109 subjects) | 17 | 2 | 3.5 | 1.8 | -1.7 |
| Glaucoma | 587 (3 or more times per week: 109 subjects) | 10 | 0 | 2.1 | 0 | -2.1 |
| Age-related macular degeneration | 593 (3 or more times per week: 109 subjects) | 5 | 0 | 1.0 | 0 | -1.0 |
| Fundus hemorrhage | 591 (3 or more times per week: 108 subjects) | 2 | 0 | 0.4 | 0 | -0.4 |
| Vitreous floaters | 593 (3 or more times per week: 109 subjects) | 2 | 0 | 0.4 | 0 | -0.4 |

In the analysis of risk of developing equilibrium dysfunction, among the analysis subjects, the number of subjects who had onset of any of equilibrium dysfunction during the investigation period was defined as the number of patients. The percentage of patients in each group was defined as incidence. Furthermore, the value obtained by subtracting the incidence in the consumers of 3 or more times per week from the incidence in the consumers of less than 3 times per week was calculated as a change in the incidence by ingestion of a Lactobacillus casei-containing composition. Tables 5 and 6 collectively show the respective results.

**[Table 5]**

| Relationship between the ingestion of Lactobacillus casei-containing composition 3 or more times per week for 5 years and the incidence of equilibrium dysfunction | | | | | | |
|---|---|---|---|---|---|---|
| | | Number of patients (subjects) | | Incidence (%) | | |
| | Number of analysis subjects (subjects) | Ingestion less than 3 times per week | Ingestion 3 or more times per week | Ingestion less than 3 times per week | Ingestion 3 or more times per week | Change in incidence by ingestion (%) |
| Dizziness syndrome | 595 (3 or more times per week: 185 subjects) | 5 | 0 | 1.2 | 0 | -1.2 |

**[Table 6]**

| Relationship between the ingestion of Lactobacillus casei-containing composition 3 or more times per week for 10 years and the incidence of equilibrium dysfunction | | | | | | |
|---|---|---|---|---|---|---|
| | | Number of patients (subjects) | | Incidence (%) | | |
| | Number of analysis subjects (subjects) | Ingestion less than 3 times per week | Ingestion 3 or more times per week | Ingestion less than 3 times per week | Ingestion 3 or more times per week | Change in incidence by ingestion (%) |
| Dizziness syndrome | 591 (3 or more times per week: 109 subjects) | 7 | 0 | 1.5 | 0 | -1.5 |
| Meniere's disease | 590 (3 or more times per week: 109 subjects) | 2 | 0 | 0.4 | 0 | -0.4 |

### 6. Results

The disease-free rate of the group in which a Lactobacillus casei-containing composition was taken 3 or more times per week for 5 years or for 10 years was significantly higher than that of the group in which the Lactobacillus casei-containing composition was taken less than 3 times per week (Figures 1 and 2, P < 0.05). That is, it was suggested that habitual ingestion of a Lactobacillus casei-containing composition significantly inhibits the decrease in the disease-free rate.

It was confirmed that the incidence of eye diseases of the group in which a Lactobacillus casei-containing composition was taken 3 or more times per week for 10 years was reduced compared to that of the group in which a Lactobacillus casei-containing composition was taken less than 3 times per week. That is, it was suggested that habitual ingestion of a Lactobacillus casei-containing composition reduces the incidence of eye diseases.

It was confirmed that the incidence of equilibrium dysfunction of the group in which a Lactobacillus casei-containing composition was taken 3 or more times per week for 5 years or for 10 years was reduced compared to that of the group in which the Lactobacillus casei-containing composition was taken less than 3 times per week. That is, it was suggested that habitual ingestion of a lactic acid bacterium-containing composition reduces the onset of equilibrium dysfunction.

## Claims

1. An agent comprising a lactic acid bacterium as an active ingredient for use in reducing the risk of developing an eye disease selected from the group consisting of age-related macular degeneration, fundus hemorrhage, and vitreous floater, or dizziness syndrome as an equilibrium dysfunction;
wherein the agent inhibits a decrease in a disease-free rate, and
wherein the lactic acid bacterium is Lactobacillus casei YIT 9029 (FERM BP-1366).

2. The agent for use according to claim 1, wherein the agent is taken before disease onset, wherein the agent is taken 3 or more times per week, wherein the agent is continuously taken for 5 or more years, and/or wherein the agent is continuously taken for 10 or more years.

3. A food or drink or a food or drink composition comprising a lactic acid bacterium as an active ingredient for use in reducing the risk of developing an eye disease selected from the group consisting of age-related macular degeneration, fundus hemorrhage, and vitreous floater, or dizziness syndrome as an equilibrium dysfunction;
wherein the food or drink or the food or drink composition inhibits a decrease in a disease-free rate, and
wherein the lactic acid bacterium is Lactobacillus casei YIT 9029 (FERM BP-1366).

4. The food or drink or the food or drink composition for use according to claim 3, wherein the food or drink is a fermented food or drink.

## Patentansprüche

1. Mittel, das ein Milchsäurebakterium als Wirkstoff enthält, zur Verwendung bei der Verringerung des Risikos, eine Augenkrankheit, die aus der Gruppe ausgewählt ist, die aus altersbedingter Makuladegeneration, Fundusblutung und Glaskörperfloater besteht, oder Schwindelsyndrom als Gleichgewichtsstörung zu entwickeln;
wobei das Mittel eine Abnahme der krankheitsfreien Häufigkeit hemmt, und
wobei das Milchsäurebakterium Lactobacillus casei YIT 9029 (FERM BP-1366) ist.

2. Das Mittel zur Verwendung nach Anspruch 1, wobei das Mittel vor Ausbruch der Krankheit eingenommen wird, wobei das Mittel 3 oder mehr Mal pro Woche eingenommen wird, wobei das Mittel kontinuierlich über 5 oder mehr Jahre eingenommen wird und/oder wobei das Mittel kontinuierlich über 10 oder mehr Jahre eingenommen wird.

3. Nahrungsmittel oder Getränk oder Nahrungsmittel- oder Getränkezusammensetzung umfassend ein Milchsäurebakterium als Wirkstoff zur Verwendung bei der Verringerung des Risikos, eine Augenkrankheit ausgewählt aus der Gruppe bestehend aus altersbedingter Makuladegeneration, Fundusblutung und Glaskörpertrübung oder von Schwindelsyndrom als Gleichgewichtsstörung zu entwickeln;
wobei das Lebensmittel oder Getränk oder die Lebensmittel- oder Getränkezusammensetzung eine Abnahme der krankheitsfreien Häufigkeit hemmt, und
wobei das Milchsäurebakterium Lactobacillus casei YIT 9029 (FERM BP-1366) ist.

4. Das Lebensmittel oder Getränk oder die Lebensmittel- oder
Getränkezusammensetzung zur Verwendung gemäß Anspruch 3, wobei das Lebensmittel oder Getränk ein fermentiertes Lebensmittel oder Getränk ist.

## Revendications

1. Agent comprenant une bactérie d'acide lactique en tant qu'ingrédient actif destiné à réduire le risque de développer une maladie oculaire sélectionnée parmi le groupe constitué d'une dégénérescence maculaire liée à l'âge, d'une hémorragie du fond de l'œil et de corps flottants, ou un syndrome de vertiges en tant que dysfonctionnement de l'équilibre ;
dans lequel l'agent inhibe une diminution d'un taux sans maladie, et
dans lequel la bactérie d'acide lactique est Lactobacillus casei YIT 9029 (FERM BP-1366).

2. Agent pour une utilisation selon la revendication 1, dans lequel l'agent est pris avant l'apparition de la maladie, dans lequel l'agent est pris 3 fois ou plus par semaine, dans lequel l'agent est pris en continu pendant 5 ans ou plus, et/ou dans lequel l'agent est pris en continu pendant 10 ans ou plus.

3. Aliment ou boisson ou composition d'aliment ou de boisson comprenant une bactérie d'acide lactique en tant qu'ingrédient actif destiné à être utilisé pour réduire le risque de développer une maladie oculaire sélectionnée parmi le groupe constitué d'une dégénérescence maculaire liée à l'âge, d'une hémorragie du fond de l'œil et de corps flottants, ou un syndrome de vertiges en tant que dysfonctionnement de l'équilibre ;
dans lequel l'aliment ou la boisson ou la composition d'aliment ou de boisson inhibe une diminution d'un taux sans maladie, et
dans lequel la bactérie d'acide lactique est Lactobacillus casei YIT 9029 (FERM BP-1366).

4. Aliment ou boisson ou composition d'aliment ou de boisson pour une utilisation selon la revendication 3, dans lequel l'aliment ou la boisson est un aliment ou une boisson fermenté(e).
